# EUROPEAN PATENT APPLICATION

(11) **EP 1 941 886 A1**
(43) Date of publication of application: **09.07.2008**
(21) Application number: 06291767.9
(22) Date of filing: 13.11.2006
(51) Int. Cl.: A61K 31/567, A61K 31/5575

(54) **Blister pack of mifepristone and misoprostol**

(71) Applicant: Exelgyn, 75007 Paris (FR)
(72) Inventor: Paris, Jean-Pierre, 78120 Sonchamps (FR)
(74) Representative: Hirsch & Associés

(57) **Abstract**

The invention provides an article of manufacture comprising (i) a packaging material and (ii) a pharmaceutical composition containing a safe and therapeutically effective amount of mifepristone or a pharmaceutically acceptable derivative thereof, and (iii) a safe and therapeutically effective amount of misopristol or a pharmaceutically acceptable derivative thereof.

## Description

### FIELD OF THE INVENTION

The invention relates to a blister pack containing a unit dosage of a mifepristone composition and two separate dosages of a misopristol composition to be taken between 24 and 72 hours later.

### BACKROUND OF THE INVENTION

Mifepristone, also known as RU486 has the chemical formula 11β-[p-(Dimethylamino)phenyl]-17β-hydroxy-17-(1-propynyl)estra-4,9-dien-3-one, and is an important antiprogestin that effectively and safely terminates early pregnancy, when used in conjunction with a synthetic prostaglandin. During early pregnancy, the endometrium is dependent upon progestagenic support from the corpus luteum. Mifepristone acts as a competitive inhibitor at the progesterone receptor and thus removes this basis for support. In addition, it sensitises the myometrium to the contraction-inducing activity of prostaglandins. Thus, the synthetic prostaglandin is administered at a separate time (e.g. 12-72 hours following the mifepristone) and causes the onset of uterine contractions and thus expulsion of the foetus. The most commonly used synthetic prostaglandin used to this purpose is misopristol, methyl7-[3-hydroxy-2- (4-hydroxy-4-methyl-oct-1-enyl) -5-oxo-cyclopentyl]heptanoate, a synthetic prostaglandin E₁ (PGE₁) analogue.

Misopristol is commercialised in a dose of 200µg under the name Gymiso^{®}, and also under the trade name Cytotec^{®}. Mifepristone is commercially available in a dose of 3 tablets of 200mg under the trade name Mifegyne^{®}. Thus, currently, the two drugs are sold separately.

In the administration of mifepristone and misopristol for the non-surgical termination of pregnancy, it is very important the mistopristol is taken within the correct time frame after taking the mifepristone for the treatment to work efficiently. In France the mifepristone/misoprostol regimen is approved up to 49 days' gestation. Women are required to orally take 600 mg of mifepristone (Mifegyne^{™}). Thirty-six to forty eight hours later, women are required to either orally take 400µg (micrograms) of misoprostol. This is usually in the form of two dosages of 200µg, at 36 and 48 hours respectively. Ten to fourteen days after taking mifepristone, the patient is required to return for a follow-up visit to determine whether the pregnancy has been terminated. The research that the FDA reviewed for approval of the regimen in the USA was based on the original French regimen, developed more than a decade ago. The mifepristone/misoprostol regimen is approved up to 49 days' gestation. The FDA approved regimen specifies that a woman orally take 600mg of Mifeprex^{™} and 400µg (micrograms) of misoprostol two days later (orally). Approximately fourteen days after taking mifepristone, the patient is required to return for a follow-up visit to determine whether the pregnancy has been terminated. In both France and the USA currently the patient is required to take the misopristol at the doctor's office or clinic, however there are now a large amount of evidence that also supports the safety, efficacy, and acceptability of home-administration of misoprostol. Several professional organization guidelines incorporate this regimen modification (reference http://www.medicationabortion.com/mifepristone/index.html).

The present invention aims to provide a mifepristone/misopristol administration system that is not only effective and safe but is also ensures a maximum of patient compliance, particularly with regard to the timing of the taking of the mispristol, which is particulary pertinent to cases where the patient takes the misopristol at home. Blister packs are well known forms in the art of pharmacy.

WO-A-03/075927 discloses a pharmaceutical combination consisting of an effective amount of artesunate and mefloquine prepacked for the treatment of malaria, in a single blister pack divided into 3 daily units, and the daily units are preferably distinguished by different colours. In this document the authors report a study by Tin Shwe et al. showing improved compliance of the patients using blister packs when taking a complex dosage regimen (WHO Bulletin OMS Vol 76 Suppl. 1 1998, p. 35-41).

WO-A-98/18477 discloses a blister pack containing lamivudine, zidovudine and a pharmaceutically acceptable glidant to be administered, for the treatment of HIV.

Currently mifepristone and misopristol are sold separately. Given the rather stressful situation that the patient seeking a non medical abortion is in, and the tendency to self administer the drugs at home rather than in the doctor's office, there is a need to provide an administration system adapted to the combined dosages of these two drugs that is safe and effective and minimises the risk of error, the consequences of which are serious.

### SUMMARY OF THE INVENTION

The invention thus provides an article of manufacture comprising:
(i) a packaging material and
(ii) a pharmaceutical composition containing a safe and therapeutically effective amount of mifepristone or a pharmaceutically acceptable derivative thereof;
(iii) a safe and therapeutically effective amount of misopristol or a pharmaceutically acceptable derivative thereof.

According to one embodiment, the article further comprises a brochure containing product information.

In another embodiment, the packaging material is a unit dose blister pack.

In another embodiment, the blister pack contains separate subunits each subunit containing the mifepristone or misopristol dosages to be taken at any one time.

In another embodiment, the subunits are labeled with a number corresponding to the hour at which each dosage should be taken.

In yet another embodiment, the subunits are also labeled with different colours.

In another embodiment, the blister pack is divided into three subunits, wherein the first subunit contains mifepristone and is labeled 0, the second subunit contains misopristol and is labeled with a number corresponding to the number of hours after the mifepristone administration at which the first misopristol dosage should be taken, and the third subunit contains misopristol and is labeled with a number corresponding to the number of hours after the mifepristone administration at which the second misopristol dosage should be taken.

In yet another embodiment, the blister pack is divided into two subunits, wherein the first subunit contains mifepristone and is labeled 0, the second subunit contains misopristol and is labeled with a number corresponding to the number of hours after the mifepristone administration at which the misopristol dosage should be taken.

The invention aims at providing a blister pack containing safe and effective amounts of mifepristone and misopristol, or pharmaceutically acceptable derivatives thereof, to be used for the non medical termination of pregnancy.

The times T0, T1 and, if appropriate, T2, at which time the mifepristone, the first and second dosages of misopristol, respectively, should be taken, are clearly indicated on the blister pack. The values of T1 and T2 will vary according to the regimen approved by the drug regulatory body governing any given country. The patient or doctor using the blister pack is at minimal risk of mixing up the two medicaments to be taken, forgetting to take one of the dosages of misopristol, or taking one or both dosages of misopristol at the wrong time with respect to the mifepristone. The risk of these errors of greater when the patient takes the treatment at home, thus the blister pack of the invention provides a great advantage in this respect.

### BRIEF DESCRIPTION OF THE DRAWINGS

In the drawings:
- Figure 1a is an illustration of a single blister pack for the administration in France of Mifepristone (three times 200mg) at T=0, and misopristol 2 times 200µg at T2=36h and T3=48h respectively.
- Figure 1b is an illustration of another dosage regimen where the dosage of mifepristone is in one tablet of, e.g. 400mg.
- Figure 2a is an illustration of a single blister pack of Mifepristone (three times 200mg) at T=0, and misopristol (two times 200µg) at T2=48h.
- Figure 2b, as in Fig. 1b, is an illustration where the mifepristone may be administered as one dose of e.g. 400mg, in which case there is only one mifepristone blister compartment.

### DETAILED DESCRIPTION OF EMBODIMENTS OF THE INVENTION

The invention relates to a blister pack containing safe and effective amounts of mifepristone and misopristol, or pharmaceutically acceptable derivatives thereof, to be used for the non-medical termination of pregnancy. The blister pack is divided into units, each unit being labelled with times T0 (always zero), T1 (for example 36 hours) and, if appropriate, T2 (for example 48 hours), at which time the mifepristone, the first and second dosages of misopristol, respectively, should be taken. The blister pack units may be colour differentiated in addition to the dosage timing labels. According to the invention the values of T1 and T2 labelling the blister pack may vary according to the approved dosage regimen of misopristol in the given country. In general, T1 and T2 each have values that range from 24 to 72 hours, T2 is present always being greater than T1.

The blister packs of the invention may be fabricated using standard means and technology suitable for medicaments such as mifepristone and misopristol.

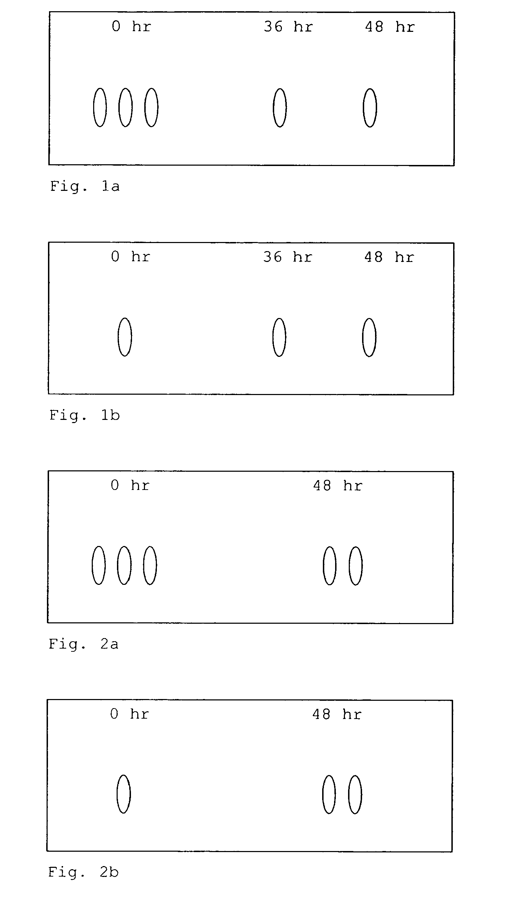

In one embodiment, as shown in fig. 1a the blister pack is divided into three units, the first containing Mifepristone (three blisters each containing 200mg) to be taken at T=0 (T=0 clearly indicated on the first blister packet unit), the second unit containing a first dosage of misopristol (e.g. 200µg) to be taken 36 hours after the mifepristone (T2=36h being clearly indicated on the second unit) and the third unit containing a second unit dosage (e.g. 200µg) of misopristol to be taken 48 hours after the mifepristone (T3=48h being indicated on the third unit).

In another embodiment as shown in fig. 1b the first unit of the blister pack contains one blister containing one single tablet of e.g. 400mg mifepristone.

In yet another embodiment as shown in fig. 2a the blister pack is divided in two units, the first containing either three blisters of 200mg each mifepristone, this first unit being clearly labelled T=0, and the second unit contains two blisters, each containing 200µg misopristol, this second unit being labeled T2=48h, indicating that the two misopristol tablets should be taken 48 hours after the mifepristone.

In another embodiment as shown in fig. 2b the first unit of the blister pack contains one blister containing one single tablet of e.g. 400mg mifepristone.

In yet another embodiment, misopristol is available as single unit, and this e.g. for the US.

The tablets of mifepristone and misopristol contained in the blister pack of the invention are galenic forms comprising active ingredients (mifepristone and misopristol) and excipients. Excipient includes any of the following components binder, diluent, disintegrating agent, lubricant and anti-static agent and diluent and optionally other auxiliary agents, such as surfactants, as is known in the art.

## Claims

1. An article of manufacture comprising
(i) a packaging material and
(ii) a pharmaceutical composition containing a safe and therapeutically effective amount of mifepristone or a pharmaceutically acceptable derivative thereof;
(iii) a safe and therapeutically effective amount of misopristol or a pharmaceutically acceptable derivative thereof.

2. An article of manufacture of claim 1 comprising a brochure containing product information.

3. An article of manufacture of claim 1 or 2 wherein the packaging material is a unit dose blister pack.

4. The blister pack of claim 3 wherein it contains separate subunits each subunit containing the mifepristone or misopristol dosages to be taken at any one time.

5. The blister pack of claim 4 wherein the subunits are labeled with a number corresponding to the hour at which each dosage should be taken.

6. The blister pack of claims 4 or 5 wherein the subunits are also labeled with different colours.

7. The blister pack of any one of claims 4, 5 or 6 which is divided into three subunits, wherein the first subunit contains mifepristone and is labeled 0, the second subunit contains misopristol and is labeled with a number corresponding to the number of hours after the mifepristone administration at which the first misopristol dosage should be taken, and the third subunit contains misopristol and is labeled with a number corresponding to the number of hours after the mifepristone administration at which the second misopristol dosage should be taken.

8. The blister pack of any one of claims 4, 5 or 6 which is divided into two subunits, wherein the first subunit contains mifepristone and is labeled 0, the second subunit contains misopristol and is labeled with a number corresponding to the number of hours after the mifepristone administration at which the misopristol dosage should be taken.
